# EUROPÄISCHE PATENTSCHRIFT

(11) **EP 2 293 776 B1**
(45) Veröffentlichungstag und Bekanntmachung des Hinweises auf die Patenterteilung: **02.03.2016**
(21) Anmeldenummer: 09768601.8
(22) Anmeldetag: 22.06.2009
(51) Int. Cl.: A61K 9/00, C07K 14/75

(54) **LAGERSTABILES, FUNKTIONELL INTAKTES VIRUS-INAKTIVIERTES FIBRINOGEN**
FIBRINOGEN BEING FUNTIONALLY INTACT , STORAGE STABLE AND VIRUS-INACTIVATED
FIBRINOGEN ÉTANT FONTIONELLEMENT INTACT, STABLE AU STOCKAGE ET INACTIVÉ DE VIRUS

(30) Priorität: 23.06.2008 US 74830 P
(43) Veröffentlichungstag der Anmeldung: 16.03.2011
(73) Patentinhaber: Bio & Bio Licensing SA, 1143 Luxembourg (LU)
(72) Erfinder: EIBL, Johann, A-1180 Wien (AT)
(74) Vertreter: Schwarz, Albin
(86) Internationale Anmeldenummer: PCT/AT2009/000248
(87) Internationale Veröffentlichungsnummer: WO 2009/155626

(56) Entgegenhaltungen:
- EP-A1- 1 393 741
- EP-A2- 0 345 246
- EP-A2- 1 568 709
- WO-A2-00/71153
- WO-A2-2004/054607
- DE-A1- 10 261 126
- SILVER F H ET AL: "Preparation and use of fibrin glue in surgery" BIOMATERIALS, ELSEVIER SCIENCE PUBLISHERS BV., BARKING, GB, Bd. 16, Nr. 12, 1. Januar 1995 (1995-01-01), Seiten 891-903, XP004032908 ISSN: 0142-9612

## Beschreibung

Die Erfindung betrifft ein lagerstabiles, funktionell intaktes Fibrinogen, ein Fibrinogenkonzentrat und ein fibrinogenhältiges Arzneimittel. Die Erfindung betrifft ferner ein Verfahren zur Herstellung einer Fibrinogenlösung durch Abtrennung von Verunreinigungen aus einer wässerigen Lösung, welche funktionell intaktes Fibrinogen enthält.

Bei Verletzungen von Blutgefäßen kommt es zu mehr oder weniger stark ausgeprägten Blutungen, die durch die Gerinnung des Blutes in den verletzten Gefäßen zum Stillstand gebracht werden. Blut kann aber auch in nicht verletzten Blutgefäßen durch andere Gefäßschädigungen gerinnen und durch die dabei entstehenden Thromben die Blutzirkulation lokal unterbrechen. Für die Gerinnung des Blutes sind Thrombin, das lösliches Fibrinogen in unlösliches Fibrin überführt, sowie aktivierte Plättchen verantwortlich. (Brummel KE et al: An Integrated Study of Fibrinogen during Blood Coagulation. J B Chem 1999; 274(32):22862-22870. Blombäck B: Fibrinogen and Fibrin-Proteins with Complex Roles in Hemostasis and Thrombosis. Thromb Res 1996; 83(1):1-75).

Thrombin spaltet enzymatisch von intaktem Fibrinogen die Fibropeptide A und B ab, wobei sich lösliche Fibrinmonomere bilden. Diese löslichen Fibrinmonomere aggregieren zu noch löslichen Fibrinmonomerkomplexen, die weiter zu unlöslichem Fibrin aggregieren. Geringe Thrombinmengen können nur eines der beiden im Fibrinmolekül enthaltenen Fibropeptide A abspalten, sodass es dann zur Bildung von α-Profibrin kommt (Shainoff JR et al: Fibrin precursors, intermediaries for hemostasis in the clot war. Thromb Res 2002; 105: 3-13).

Zur enzymatischen Wirkung von Thrombin auf Fibrinogen, das zur Abspaltung des Fibrinopeptids A und nachfolgend zur Abspaltung des Fibrinopeptids B fahrt, muss sich Thrombin an die Spaltstellen der Fibrinopeptide im Fibrinogenmolekül binden. Darüberhinaus wird aber Thrombin noch an zwei weiteren Stellen an das Fibrinogenmolekül gebunden, wo es keine Spaltung verursachen kann. Dieses so gebundene Thrombin ist enzymatisch unwirksam und steht durch seine Bindung auch nicht für andere enzymatische Reaktionen zur Verfügung. Die Eigenschaft des Fibrinogens, Thrombin auch an Stellen im Molekül zu binden, wo es keine enzymatische Aktivität ausüben kann, wird als AntithrombinI-Aktivität bezeichnet (Mosesson M: Antithrombin I. Inhibition of thrombin generation in plasma by fibrin formation. Thromb Haemost 2003; 89:9-12. Meh DA: Identification and Characterization of the Thrombin Binding Sites on Fibrin. J B Chem 1996; 271(38):23121-23125).

Fibrinogenkonzentrate für pharmazeutische Zwecke werden sowohl zur topischen Applikation für Blutstillung und Gewebeklebung, als auch für intravenöse Infusionen zur Behandlung von A-, Hypo- und Dysfibrinogenämie und Verbrauchskoagulopathie verwendet. Solche Arzneimittel sind in tiefgefrorener Form oder gefriergetrocknet erhältlich und für 18 bis 36 Monate lagerfähig. Beide Formen weisen Nachteile auf. Die tiefgefrorenen Fibrinogenlösungen müssen tiefgekühlt gelagert und transportiert werden, und der nachfolgende Auftauprozess nimmt geraume Zeit in Anspruch. Die gefriergetrockneten Fibrinogenkonzentrate können zwar bei Kühlschranktemperatur gelagert werden, aber die erforderliche lange Rekonstitutionzeit verhindert eine prompte Anwendung. Sowohl die aufgetaute wie auch die rekonstituierte Fibrinogenlösung führt bei mehrtägiger Lagerung im Kühlschrank zur Gelbildung und wird dadurch unverwendbar. Gleichzeitig erfolgt auch ein fibrinogenolytischer Abbau des Fibrinogens, der zur Verminderung des gerinnbaren Proteins führt (Marder VJ et al: High molecular weight derivatives of human fibrinogen produced by plasmin. I. Physicochemical and immunological characterization. J Biol Chem 1969; 244(8):2111-9).

In der Vergangenheit wurden verschiedenste Methoden angewandt, wie z.B. chromatographische Reinigung oder andere Fraktionierungsschritte, die zwar zu besser gereinigten Fibrinogenpräparationen führten, jedoch die erwähnten Nachteile nach wie vor aufwiesen (Kannelos J: Isolation of Fibrinogen by Affinity Chromatography. WO 97/26280; 1997. Hock J et al: Stable Fibrinogen Solution. US 6,277,961 B1; 2001. Metzner H: Reinigung von Fibrinogen. EP 1 568 709 A2; 2005. Dazey B: Verfahren zur Herstellung von hochreinem Fibrinogen. DE 693 03 941 T2; 1993. Burnouf T: Chromatographische Trennung von Plasmaproteinen, insbesondere von Faktor VIII, von Willebrand Faktor, von Fibronektin und von Fibrinogen. DE 689 22 358 T3; 1990. Nogre M: Process for separating proteins fibrinogen, factor XIII and biological glue from a solubilized plasma fraction and for preparing lyophilised concentrates of said proteins. AU 2006202680 AT; 2005).

Versuche, durch Erhöhung der Salzkonzentration oder durch Zusatz chaotroper Agenzien eine höhere Lagerstabilität und eine kürzere Rekonstitutionszeit von gefriergetrocknetem Fibrinogen zu erreichen, schlugen wegen der erforderlichen hohen Osmolalität bzw. auftretenden Toxizität fehl. (Metzner H et al: Stabilized Protein Preparations for a Tissue Adhesive. US 6,447,774 B1; 2002. Chabbat J: Fluid Biological Glue. WO 91/19519; 1991. Metzner H et al: Stabilisierte Proteinzubereitung für einen Gewebekleber. DE 198 53 033 A1; 2000).

Hochgereinigte Fibrinogenpräparationen, die praktisch nur mehr gerinnbares Protein enthalten, können, insbesondere nach längerer Lagerung, nur mehr etwa zur Hälfte aus funktionell intaktem Fibrinogen bestehen. Der Anteil des nicht intakten Fibrinogens im gerinnbaren Protein besteht aus Profibrin, Fibrinmonomeren und Fibrinmonomerkomplexen sowie noch gerinnbaren Fibrinogenabbauprodukten.

Während der Herstellung und Lagerung von Fibrinogenpräparationen kommt es in unterschiedlichem Ausmaß durch enzymatische Einwirkungen auf Kosten des funktionell intakten Fibrinogens zur weiteren Bildung von Fibrinogenabbauprodukten, und damit zur Qualitätsverschlechterung einer Fibrinogenpräparation, insbesondere zu einer schlechteren Rekonstitutierbarkeit von gefriergetrocknetem Fibrinogen.

### Aufgabe der Erfindung

Gefriergetrocknete, fibrinogenhältige Arzneimittel sollten schnell, d.h. in kurzer Zeit, rekonstituierbar sein und diese schnelle Rekonstituierbarkeit während ihrer gesamten Laufzeit bewahren. Ferner sollten die durch Rekonstitution mit entsprechenden Lösungsmitteln erhaltenen Fibrinogenlösungen bei Kühlschranktemperatur flüssig und ohne wesentliche Steigerung der Viskosität eine Woche lang haltbar sein, ohne dass gerinnbares Protein verloren geht.

Das in den Ausgangsmaterialien vorliegende gerinnbare Protein ist bereits ein Gemisch aus intaktem Fibrinogen, Profibrin, Fibrinmonomeren, Fibrinmonomerkomplexen und auch fibrinogenolytischen Abbauprodukten. Die Abbauprodukte des intakten Fibrinogens entstehen durch Enzyme, die bereits im Plasma und in den Plasmarohfraktionen meist in unterschiedlichem Ausmaß vorhanden sind. Es ist daher erforderlich, Herstellungsmethoden zu entwickeln, bei denen störende enzymatische Aktivitäten während der Produktion unterbunden und Abbauprodukte des intakten Fibrinogens, insbesondere Fibrinmonomere und Fibrinmonomerkomplexe, weitgehend abgereinigt werden, da diese eine schnelle Rekonstituierbarkeit des gefriergetrockneten Fibrinogens verhindern. Eigene Untersuchungen haben gezeigt, dass die Stabilität von Fibrinogen von dessen Gehalt an Enzymen, die Fibrinogen als ihr Substrat erkennen, stark abhängig sein kann.

Um größere Verluste an intaktem Fibrinogen bei der Herstellung zu vermeiden, ist es vorteilhaft, alle enzymatischen Prozesse, die zu einer Veränderung des intakten Fibrinogens führen, so weit wie möglich zu unterbinden. Solche enzymatischen Prozesse werden durch Enzyme, die Fibrinogen als ihr Substrat erkennen, verursacht, die bereits vorhanden sind bzw. sich während des Herstellungsprozesses aus ihren Zymogenen bilden. Besonders bei Verfahrensschritten, die bei Zimmertemperatur oder erhöhter Zimmertemperatur durchgeführt werden, wie zum Beispiel die S/D Virusinaktivierung, muss darauf geachtet werden, dass es weder zur Bildung solcher Enzyme aus Zymogenen, noch zur Einwirkung dieser Enzyme auf Fibrinogen kommt. Ein weiteres Erfordernis ist die Stabilität von funktionell intaktem Fibrinogen in einem Arzneimittel, dessen Fibrinogenkonzentration bei der Anwendung zwischen 0,3 und 10% liegt.

Flüssige, fibrinogenhältige Arzneimittel sollten wie gefriergetrocknetes Fibrinogen im Kühlschrank für einen Zeitraum von 18-36 Monaten lagerfähig sein. Während der Dauer der Lagerung soll es zu keiner wesentlichen Zunahme der Viskosität und zu keinem Verlust an funktionell intaktem Fibrinogen kommen.

### Erfindungsgemäße Aufgabenlösung

Das erfindungsgemäße Fibrinogen kann aus einer aus einer wässerigen Lösung hergestellt werden, welche funktionell intaktes Fibrinogen enthält und mit Profibrin und/oder Fibrinmonomeren und/oder Fibrinmonomerkomplexen und/oder Fibrinspaltprodukten verunreinigt ist, indem die Verunreinigungen mit einem nicht denaturierenden Fällungsmittel bei einer Temperatur zwischen -4° bis +4° C und einer Ca-Ionenaktivität, die nicht höher ist, als jene einer 1.000 µM CaCl₂-Lösung, gefällt und durch Filtration oder Zentrifugation aus der wässerigen Lösung abgetrennt werden. Das Filtrat bzw. Zentrifugat enthält das erfindungsgemäße Fibrinogen in gelöster Form.

Unter der Bezeichnung "nicht denaturierendes Fällungsmittel" wird ein Fällungsmittel verstanden, welches Fibrinogen nicht denaturiert.

Ein wesentliches Merkmal des erfindungsgemäßen Verfahrens ist somit, die Ca-Ionenaktivität abzusenken. Es hat sich gezeigt, dass dann ein Fibrinogen gewonnen werden kann, welches nach Gefriertrocknung schnell wieder rekonstituerbar ist. Im Sinne der vorliegenden Beschreibung und der vorliegenden Patentansprüche liegt eine schnelle Rekonstituierbarkeit dann vor, wenn die Rekonstitutionszeit des gefriergetrockneten Fibrinogens bei Raumtemperatur maximal 10 Minuten ist, wie unten noch ausführlich beschrieben ist. Demgegenüber weisen die gefriergetrockneten Fibrinogenkonzentrate des Standes der Technik erheblich längere Rekonstitutionszeiten von etwa 20 bis 30 Minuten auf.

Als Ausgangsmaterial haben sich fibrinogenhältige Plasmarohfraktionen bewährt, die bei Temperaturen zwischen -4° und + 4° C hergestellt werden können. In diesem Temperaturbereich sind Enzyme, die Fibrinogen als ihr Substrat erkennen, gering wirksam. In industriellem Maßstab können solche fibrinogenhältigen Plasmarohfraktionen durch Alkoholfällung von Plasma oder von Kryoüberstand als Cohn-Fraktion-I oder durch Kältefällung als Kryopräzipitat gewonnen werden. Die fibrinogenhältigen Plasmarohfraktionen Cohn-Fraktion-I und Kryopräzipitat sind in Wasser für Injektionszwecke gut löslich, und solche fibrinogenhältigen Lösungen können bis zur weiteren Verarbeitung tiefgefroren gelagert werden.

Bei nicht sorgfältig gewonnenem Plasma, insbesondere wenn es über lange Zeit tiefgefroren gelagert wurde, sind Fibrinmonomere und Fibrinmonomerkomplexe in größerem Ausmaß vorhanden. Wenn aus diesem Plasma Kryopräzipitate gewonnen werden, wird ein hoher Anteil solcher Fibrinmonomere präzipitiert, sodass es dann zweckmäßig erscheint, nur das noch im Kryoüberstand vorhandene Fibrinogen durch Alkoholfällung zur weiteren Reinigung zu gewinnen.

Eine bevorzugte Ausführungsform des erfindungsgemäßen Verfahrens ist dadurch gekennzeichnet, dass die wässerige Lösung bei der Fällung eine elektrische Leitfähigkeit von ≤ 500 µS/cm aufweist, vorzugsweise bei einer Temperatur zwischen 0° und 2°C. Es hat sich gezeigt, dass die Verunreinigungen unter diesen Bedingungen leichter und in höherem Ausmaß aus der wässerigen Lösung gefällt werden können, sodaß das erfindungsgemäße Fibrinogen noch reiner ist.

Weiters bevorzugt ist, wenn die Ca-Ionenaktivität nicht höher ist, als jene einer 500 µM CaCl₂-Lösung, insbesondere nicht höher, als jene einer 50 µM CaCl₂-Lösung.

Besonders bevorzugt ist eine Variante des erfindungsgemäßen Verfahrens, bei der die Lösung eine Temperatur zwischen 0° und 2° C, eine elektrische Leitfähigkeit zwischen 10 bis 100 µS/cm und eine Ca-Ionenaktivität aufweist, die nicht höher ist, als jene einer 500 µM CaCl₂-Lösung.

Das in der erhaltenen Fibrinogenlösung enthaltene Fibrinogen kann durch mindestens eine weitere Fällung weiter gereinigt werden.

Mit dem erfindungsgemäßen Verfahren kann eine Fibrinogenlösung mit einem Gehalt von 0,3% bis 10% gerinnbarem Protein hergestellt werden, die bis zu 36 Monate bei einer Temperatur von 2° bis 8° C haltbar ist, durch Abreinigung von Enzymen und deren Proenzymen, die als solche bzw. im aktivierten Zustand Fibrinogen als Substrat erkennen, mit Hilfe von nicht denaturierenden Fällungsmitteln und erforderlicherweise durch Zusatz von entsprechenden virusinaktivierten, homologen Proteaseinhibitoren.

Die Fibrinogenlösung wird bevorzugt einem Verfahren zur Virusinaktivierung oder Virusabreicherung unterzogen, die bei Temperaturen über der Raumtemperatur, einer Thrombinaktivität von ≤ 0,03 IE/ml und einer Ca-Ionenaktivität, die jene einer 1.000 µM CaCl₂-Lösung nicht übersteigt, durchgeführt wird.

Die Viren können durch Nanofiltration bei Temperaturen zwischen 20° und 40°C und mit Nanofiltern einer Porenweite von maximal 20 nm, vorzugsweise 15 nm, abgereichert werden, wobei die Nanofiltration vorzugsweise so durchgeführt wird, dass es nicht zur Abspaltung von Fibrinopeptid A kommt.

Die erfindungsgemäß hergestellte Fibrinogenlösung wird zweckmäßigerweise aufkonzentriert, tiefgefroren oder gefriergetrocknet.

Beschrieben ist ferner ein Fibrinogenkonzentrat, das nach dem erfindungsgemäßen Verfahren erhältlich ist.

Beschrieben ist ferner das erfindungsgemäße Fibrinogenkonzentrat in gefriergetrockenter Form, zur Verwendung als pharmazeutischer Wirkstoff, wobei das Konzentrat mit einem geeigneten Lösungsmittel zu einer 0,3% bis 10%-igen Fibrinogenlösung rekonstituiert, mindestens 10 Tage zwischen 2° und 8°C gelagert werden kann, ohne dass der Gehalt an gerinnbarem Protein abnimmt oder eine Gelbildung erfolgt.

Das gefriergetrocknete Fibrinogenkonzentrat ist nach einer Lagerzeit von mindestens 18 Monaten bei 2° bis 8° C mit einem Lösungsmittel in weniger als 10 Minuten rekonstituierbar.

Eine bevorzugte Ausführungsform des Fibrinogenkonzentrats enthält Fibrinogen, aus dem mit Thrombin mindestens 70% Fibrinopeptid A, bezogen auf das gerinnbare Protein, abspaltbar ist.

Weitere bevorzugte Ausführungsformen des Fibrinogenkonzentrats sind dadurch gekennzeichnet,
- dass sie mindestens 1 arbiträre Einheit AT-I pro 10 mg gerinnbarem Protein enthalten, oder
- dass sie keine Partikel mit einem Durchmesser von mehr als 20 nm, vorzugsweise 15 nm, enthalten,
oder
- dass sie nach Gefriertrocknung 30 bis 180 Minuten auf eine Temperatur von 90° bis 145° C erhitzt wurden.

Darüberhinaus betrifft die Erfindung ein Arzneimittel, welches das Fibrinogenkonzentrat als Wirkstoff enthält.

Das Arzneimittel kann in einer Fertigspritze oder in einem Infusionsbeutel enthalten sein.

Die Erfindung betrifft ferner ein Verfahren zur Herstellung eines Arzneimittels, welches das gefriergetrocknete Fibrinogenkonzentrat enthält und dem basische Aminosäuren bis zur Hälfte des Gewichts an gerinnbarem Protein zugesetzt wurden, sowie Salze, um die Osmolarität zu gewährleisten, wobei das gefriergetrocknete Fibrinogenkonzentrat in einem Lösungsmittel rekonstituiert wird, das eine Ca-Salzmenge enthält, die gewährleistet, dass das rekonstituierte Produkt eine Ca-Ionenaktivität aufweist, die jene einer 5 mM Ca-Cl₂-Lösung entspricht.

Das Arzneimittel in gefriergetrockneter Form kann ferner in einem Endverbraucherbehältnis für eine Dauer von 30 bis 100 Minuten bei Temperaturen zwischen 90° und 145°C zur Inaktivierung etwaig vorhandener Viren hitzbehandelt werden.

Schließlich beschrieben ist noch die Verwendung der erfindungsgemäßen Fibrinogenlösung oder des erfindungsgemäßen Fibrinogenkonzentrates zur Herstellung eines Arzneimittels zur Behandlung von A-, Dys- und Hypofibrinogenämie, und zur Behandlung von Patienten mit Verbrauchskoagulopathie und Schock, insbesondere septischem Schock.

Im Folgendem wird die Erfindung noch näher beschrieben.

Wie bereits erwähnt, ist es für die Herstellung von Fibrinogenkonzentraten mit einem hohen Anteil an funktionell intaktem Fibrinogen wünschenswert, die Wirkungen von Enzymen, die Fibrinogen als ihr Substrat erkennen, weitestgehend zu unterbinden und ebenso die Bildung solcher Enzyme aus ihren Zymogenen. Dies wird erfindungsgemäß durch eine starke Verminderung der Ca-Ionenaktivität und die strikte Einhaltung eines Temperaturbereichs zwischen -4° und 4°C erreicht. Durch Zusatz von EDTA wird die Ca-Ionenaktivität so weit gesenkt, dass ihr Wert unter der Ionenaktivität einer 1.000 µM Ca-Cl₂-Lösung liegt. Durch fraktionierte Fällung mittels nicht-denaturierender Fibrinogenfällungsmittel, wie z.B. Glyzin, kann eine Fibrinogenfraktion mit mindestens 90% gerinnbarem Protein erhalten werden. Wenn erforderlich, kann durch mehrfache Umfällung ein Fibrinogenkonzentrat erhalten werden, aus dem, bezogen auf gerinnbares Protein, mindestens 70% Fibrinopeptid A durch Thrombin abspaltbar sind, und das nach Gefriertrocknung schnell löslich ist.

Wenn solche Fibrinogenkonzentrate in einer 1%igen Lösung hohe Thrombin- oder Kallikreinaktivität enthalten, kann mittels enzymfreier Inhibitoren die Enzymaktivität reduziert werden. Es ist zweckmäßig, virussichere Inhibitoren zuzusetzen, die, falls erforderlich, durch weitere Reinigungsschritte wieder abgetrennt werden können.

Die Virusinaktivierung kann dann durch Zusatz von Tween-80 und Trinitrobutylphosphat in den erforderlichen Mengen bei Temperaturen zwischen 28° und 30° C bis zu 10 Stunden durchgeführt werden, ohne dass sich dabei messbare Mengen Fibrinopeptid A von Fibrinogen abspalten.

Die Abreinigung des Virusinaktivierungsmittels aus dem virusinaktivierten Fibrinogenrohkonzentrat kann chromatographisch oder durch fraktionierte Fällung erfolgen. Zur Fällung können bei Temperaturen über 0° C Aminosäuren, Polyäthylenglykol und andere, nicht toxische und nicht denaturierende Fällungsmittel dienen. Weiters besteht auch die Möglichkeit, durch Alkoholfällung bei Temperaturen zwischen -3° bis - 5°C Tween-80 und Trinitrobutylphosphat zu entfernen.

Das virusinaktivierte und gereinigte Fibrinogenkonzentrat kann noch geringe Mengen an Fibrinmonomeren und Fibrinmonomerkomplexen enthalten, die durch Kältepräzipitation bei sehr niedriger elektrischer Leitfähigkeit entfernt werden können. Die Absenkung der Leitfähigkeit auf einen Wert unter 500 µS/cm und insbesondere zwischen 10 und 100 µS/cm kann durch Dialyse, Diafiltration oder auch durch Alkoholfällung in der Kälte erreicht werden.

Die Kältefällung wird bei Temperaturen zwischen -4° und +4° C durchgeführt. Der pH-Wert ist vorzugsweise zwischen 7,1 bis 7,3. Eine Erniedrigung des pH-Werts auf bis 6,5 verstärkt die Kältefällung, verschlechtert aber die Ausbeute an funktionell intaktem Fibrinogen. Durch Rühren, Vibration oder Beschallung kann die Kältefällung erhöht werden. Durch Filtration oder Zentrifugation wird das Kältepräzipitat entfernt, das im Wesentlichen aus Fibrinmonomeren und deren Komplexen besteht. Im Überstand, bzw. Filtrat, befindet sich das funktionell intakte Fibrinogen, das durch Fällung, vorzugsweise mit Alkohol unter 0° C gewonnen werden kann. Das auf diese Weise hochgereinigte Fibrinogen ist funktionell intakt und nach Gefriertrocknung schnell rekonstituierbar. Während der gesamten Herstellung muss darauf geachtet werden, dass die Ca-Ionenaktivität nicht die Ionenaktivität einer 1.000 µM CaCl₂-Lösung übersteigt.

Wenn erforderlich, können durch weitere Reinigungsschritte Proenzyme, wie Faktor XIII oder Plasminogen, entfernt werden. Für manche Anwendungen sind aber bestimmte Proenzyme erforderlich. Sie werden vorzugsweise in virussicherer Form während der Formulierung einer Fibrinogenpräparation zugesetzt.

Das hochgereinigte Fibrinogenkonzentrat kann noch immer, wenn auch in niedriger Konzentration, Enzyme enthalten, die Fibrinogen nachteilig verändern. Um diese Enzyme zu inhibieren, werden vorteilhaft virusinaktivierte, atoxische, enzymfreie homologe Proteaseinhibitoren zugesetzt, wie AT-III, Heparin-Cofaktor-II und C₁-Esterase-Inhibitor. Die so erhaltenen Fibrinogenkonzentrate, vorzugsweise mit einer Ca-Ionenaktivität, die unter der einer 50 µM CaCl₂-Lösung liegt, können in flüssigem Zustand als Wirkstoff oder formuliert in einem Konzentrationsbereich von Fibrinogen zwischen 0,3 und 10% bei 2° bis 8° C mindestens 18 Monate gelagert werden.

Fibrinogenlösungen, die keine Thrombinaktivität mehr besitzen, können dennoch Thrombin enthalten, das an Stellen des Fibrinogenmoleküls gebunden ist, die durch Thrombin nicht aufgespalten werden können. Durch Zusatz von nicht-toxischen chaotropen Substanzen kann die Fibrinogen-Thrombinbindung gesprengt und Thrombin von Fibrinogen abgetrennt werden. Das so gereinigte Fibrinogen weist eine hohe AT-I-Wirkung auf. Durch Umfällung werden die chaotropen Substanzen wieder entfernt.

Das potentielle Risiko einer Virusübertragung durch menschliche Blutprodukte kann durch Nanofiltration reduziert werden. Fibrinogenlösungen, die keine oder nur sehr geringe Mengen Fibrinmonomerkomplexe enthalten, können durch Nanofilter mit einer Porengröße von 15 bis 20 nm gut filtriert werden, wobei eine Erhöhung der Temperatur der zu filtrierenden Lösung über die Raumtemperatur die Filtrationsgeschwindigkeit beschleunigt. Praktisch enzymfreie Fibrinogenlösungen können bis zu einer Temperatur von 40°C nanofiltriert werden, und dadurch kann eine mindestens doppelte Erhöhung der Filtrationsgeschwindigkeit erreicht werden.

Eine weitere Risikoverminderung einer Virusübertragung wird durch Erhitzen von gefriergetrocknetem Fibrinogen auf Temperaturen zwischen 90° und 145°C für eine Dauer von 30 bis 180 Minuten erreicht. Diese Erhitzung kann sowohl in gefriergetrocknetem Bulkmaterial als auch in Endverbraucherbehältern erfolgen.

Die erfindungsgemäßen Verbesserungen der Fibrinogengewinnung ermöglichen die Herstellung von fibrinogenhältigen Arzneimitteln, die einfacher gelagert und transportiert werden können, und eine raschere und breitere therapeutische Anwendung erschließen.

Beschrieben ist daher auch ein gefriergetrocknetes Fibrinogenkonzentrat als pharmazeutischen Wirkstoff, das mit einem geeigneten Lösungsmittel zu einer 0,3% bis 10% Fibrinogenlösung rekonstituiert, und 10 Tage zwischen 2° und 8° C gelagert werden kann, ohne dass der Gehalt an gerinnbarem Protein abnimmt oder eine Gelbildung erfolgt.

Eine weitere Ausführungsform des Fibrinogenkonzentrats ist dadurch gekennzeichnet, dass dessen Gehalt an gerinnbarem Protein aus funktionell intaktem Fibrinogen und weniger als 10%, vorzugsweise weniger als 3%, Profibrin bzw. Fibrinmonomeren besteht, virusinaktiviert ist und gefriergetrocknet innerhalb von 36 Monaten bei 2° bis 8° C in Mengen von Lösungsmitteln, die eine 0,3 bis 10%ige Lösung von gerinnungsfähigem Protein ergeben, schnell löslich ist, wobei diese Lösungen bis zu zehn Tagen bei 2° bis 8° C gelagert, keine Veränderung der Viskosität und keine Abnahme des gerinnbaren Proteins erfahren.

Eine weitere Ausführungsform des Konzentrats ist dadurch gekennzeichnet, dass weder Enzyme, die Fibrinogen als Substrat erkennen, noch Proenzyme, die zu solchen Enzymen aktiviert werden können, enthalten sind und es in einer 0,3 bis 10%igen Lösung bei 2° bis 8°C mindestens 18, vorzugsweise 36, Monate haltbar ist.

Ferner betrifft die Erfindung ein Verfahren zur Herstellung von Fibrinogenkonzentraten mit einer AT-I-Aktivität von mindestens 1 arbiträren Einheit AT-I pro 10 mg gerinnbarem Protein, durch fraktionierte Fällung mit nicht denaturierenden Fällungsmitteln, nach vorheriger Trennung der Enzym-Substrat-Bindung von Thrombin mit Fibrinogen durch nicht toxische chaotrope Substanzen.

Mit den nachfolgenden Beispielen werden bevorzugte Ausführungsformen der Erfindung noch näher beschrieben.

### Beispiele

### 1. Schnell rekonstituierbares, gefriergetrocknetes Fibrinogenkonzentrat

1000 g Kryopaste oder Cohn-Fraktion-I Präzipitat werden in einer 0,01 M EDTA-Lösung, die 2 E pro ml enthält, unter ständigem Rühren bei 4° ± 1° C gelöst, um eine 1 - 2%ige Proteinlösung zu erhalten. In diese Lösung wird wasserfreies Glyzin eingerührt, bis der Gehalt 6 g/v% beträgt. Nach 10-stündigem Rühren bei 2°C wird der entstandene Niederschlag in einem Temperaturbereich von 2° - 4°C durch Zentrifugation oder Filtration entfernt. Der Niederschlag enthält einen großen Teil der im Ausgangsmaterial enthaltenen Fibrinmonomerkomplexe und Fibrinmonomere. Der Überstand, bzw. das Filtrat, wird mit Glyzin bei einer Temperatur zwischen 2° und 4° C gesättigt und wieder 10 Stunden bei dieser Temperatur gerührt. Das dabei ausgefallene erfindungsgemäße Fibrinogen wird hochtourig in einer Sharples Zentrifuge bei einer Umdrehungszahl von etwa 17000 Umdrehungen pro Minute gewonnen und wieder in einer 1/100 M EDTA-Lösung aufgelöst, um eine etwa 1%ige Lösung an gerinnbarem Protein zu erhalten.

Das gelöste Fibrinogen wird bei einer Temperatur von 2° bis 4° C gehalten und es wird eine Probe zur Bestimmung von Thrombin, Gesamtprotein und gerinnbarem Protein entnommen.

Die Ca-Ionenaktivität der Lösung, in welcher die Fällung der Verunreinigungen vorgenommen wurde, war unter jener einer 500 µM CaCl₂-Lösung. Die Ca-Ionenaktivität wird mit Hilfe einer Ca-Elektrode bestimmt und der Wert der Ionenaktivität nach einer Kurve ermittelt, die die Abhängigkeit der mit der Elektrode gemessenen Spannung von der CaCl₂-Konzentration wiedergibt. Wenn die Ca-Ionenkonzentration zu hoch ist, wird mit einer 1/10 M EDTA-Lösung die Ca-Ionenaktivität entsprechend gesenkt.

Die Thrombinaktivität betrug weniger als 1 mE Thrombin pro ml. Die Bestimmung von Thrombin erfolgt mit dem chromogenen Substrat 2238 unter Bezugnahme auf eine Thrombinreferenzpräparation. Wenn der Thrombingehalt mehr als 1 mE Thrombin pro ml beträgt, wird durch Zusatz von AT-III und Heparin in einem Einheitenverhältnis von 1 + 3 die Thrombinaktivität inhibiert, bzw. durch Umfällung mit Glyzin.

Wenn erforderlich, kann Glyzin aus der Fibrinogenlösung durch Diafiltration weitgehend entfernt werden, ebenso auch durch Umfällung mit Alkohol unter 0°C. Zur Lagerung bis zur weiteren Verarbeitung kann die Fibrinogenlösung entweder tiefgekühlt bei -20° C gehalten oder durch Gefriertrocknung zu einem bei 4° C lagerbaren Fibrinogen verarbeitet werden, das prompt löslich ist.

### 2. Virusinaktiviertes, schnell lösliches Fibrinogenkonzentrat

Die etwa 1%ige Proteinlösung des nach Beispiel 1 hergestellten Fibrinogenkonzentrates wird mit der erforderlichen Menge Tween-80 und Trinitrobutylphosphat versetzt und 10 Stunden bei 30° C gehalten. Durch eine davor durchgeführte Probevirusinaktivierung wird sichergestellt, dass es bei der Lagerung einer Probe für die Dauer von 30 Stunden zu keiner Freisetzung von Fibropeptid A kommt. Zur Entfernung des zugesetzten Tween-80s und Trinitrobutylphosphats werden Umfällungen mit nicht-denaturierenden Fällungsmitteln, wie z.B. Glyzin, oder mit Alkohol unter 0° C bis zu einem Alkoholgehalt von 10 %, durchgeführt.

Virusinaktiviertes Fibrinogenkonzentrat kann entweder in tiefgefrorenem oder gefriergetrocknetem Zustand gelagert und darnach schnell aufgetaut bzw. schnell rekonstituiert werden.

### 3. Funktionell intaktes, virusinaktiviertes Fibrinogen mit schneller Löslichkeit

10 Liter einer nach Beispiel 2 hergestellten Lösung mit 1 % virusinaktiviertem, gerinnbarem Protein werden durch Diafiltration gegen eine 1/100 M EDTA-Lösung oder durch Umfällung mit Äthylalkohol und Lösen des Alkoholniederschlags in einer 1/00 M EDTA-Lösung auf eine elektrische Leitfähigkeit von 10 bis 100 µS gebracht. Der pH-Wert der Lösung wird auf 7,1 bis 7,3 eingestellt und die Lösung unter leichtem Rühren 10 Stunden auf einer Temperatur zwischen 2° und 4° C gehalten. Dadurch kommt es zu einer Ausfällung der in der Lösung noch vorhandenen Fibrinmonomerkomplexe und ebenso zu einer teilweisen Fällung von Fibrinmonomeren. Durch Zentrifugation bei etwa 17000 Umdrehungen pro Minute in einer Sharpless Zentrifuge wird der Niederschlag abgetrennt. Wenn der Überstand noch eine Opaleszenz aufweist, wird eine Filtration durch ein Filter mit 5 µ durchgeführt. Die so erhaltene Lösung weist einen Gehalt von mindestens 90% funktionell intaktem Fibrinogen, bezogen auf das in der Lösung vorhandene gerinnungsfähige Protein, auf. Durch Absenkung des pH-Wertes bis auf 6,5 kann der prozentuelle Gehalt an funktionell intaktem Fibrinogen noch weiter gesteigert werden, wobei allerdings eine Verringerung der Ausbeute eintritt. Die so erhaltenen Lösungen an funktionell intaktem Fibrinogen können in Bulk bis zur weiteren Verarbeitung oder zu ihrer Formulierung zu Arzneimitteln tiefgefroren oder gefriergetrocknet gelagert werden.

### 4. Funktionell intaktes Fibrinogen, virusinaktiviert, flüssig haltbar

Lösungen von funktionell intaktem Fibrinogen, wie sie nach Beispiel 3 erhalten werden, können noch immer Enzyme enthalten, die Fibrinogen enzymatisch verändern. Es ist daher erforderlich, solche Enzymaktivitäten durch atoxische, virusinaktivierte, aus menschlichem Plasma hergestellte Enzyminhibitoren zu inhibieren. Zu diesem Zweck wird die Thrombinaktivität und die Kallikreinaktivität im Fibrinogenkonzentrat bestimmt und so viel virusinaktiviertes und enzymfreies AT-III zugesetzt, bis keine Thrombinaktivität mehr nachweisbar ist. Ein Zusatz von Heparin erfolgt nicht. Um die Kallikreinaktivität zu inhibieren, ist der Zusatz eines aus menschlichem Plasma gewonnenen C₁-Esterase Inhibitors erforderlich, der keinerlei enzymatische Aktivitäten enthält und virussicher ist. Zur Bestimmung der Kallikreinaktivität wird das chromogene Substrat 2302 verwendet.

Enzymfreie Lösungen von funktionell intaktem Fibrinogen werden durch Diafiltration oder durch Fällung mit Alkohol und Lösen des Alkoholniederschlags in Konzentrationen zwischen 0,3 bis 10% hergestellt und sind bei Temperaturen zwischen 2° und 8°C haltbar.

### 5. Herstellung eines funktionell intakten, virusinaktivierten Fibrinogenkonzentrats mit hohem AT-I Gehalt

Das nach Beispiel 4 hergestellte, enzymfreie Fibrinogenkonzentrat enthält noch weiteres, an Fibrinogen gebundenes Thrombin an Stellen des Moleküls, die nicht durch Thrombin enzymatisch gespalten werden. Um die Bindung zwischen Fibrinogen und Thrombin zu lösen, werden der Lösung pro g intaktem Fibrinogen 2 g Harnstoff zugesetzt und eine Stunde lang bei 35° C leicht gerührt. Danach wird Fibrinogen mit Natriumzitrat in einem po-Bereich zwischen 7,4 bis 7,8 ausgefällt, die Fällung durch Zentrifugation gewonnen und, falls erforderlich, ein oder mehrere Male mit Natriumzitrat umgefällt, wobei der gelöste Niederschlag pro g Fibrinogen 2g Harnstoff enthält. Zum Schluss wird die Fibrinogenlösung gegen eine 1/100 M EDTA-Lösung diafiltriert, um Harnstoff und Zitrat zu entfernen. Die diafiltrierte Lösung ist bei Temperaturen zwischen 2° und 8°C lagerbar. Ebenso kann daraus gefriergetrocknetes Fibrinogen hergestellt werden.

### 6. Nanofiltriertes Fibrinogenkonzentrat

Die nach den Beispielen 2 - 5 hergestellten Fibrinogenkonzentrate können noch zur Erhöhung der Virussicherheit nanofiltriert werden, vorzugsweise mit einer Fibrinogenkonzentration zwischen 0,1 und 1%. Vor der Nanofiltration werden die fibrinogenhältigen Lösungen durch Filtration geklärt, wofür Filter von 75 nm bis 35 nm benützt werden und darauf eine Nanofiltration durch Nanofilter mit einer Porengröße von 20 nm, bzw. 15 nm erfolgt. Soweit die Fibrinogenkonzentrate keine enzymatische Aktivität aufweisen, kann die Nanofiltration zur Steigerung der Filtrationsgeschwindigkeit bei Temperaturen bis zu 40° C durchgeführt werden.

### 7. Hitzebehandlung von gefriergetrocknetem, schnell löslichem, funktionell intaktem, virusinaktiviertem Fibrinogen

Gefriergetrocknetes Fibrinogenkonzentrat wird pulverisiert und durch ein Sieb mit einer Maschenweite von 0,5 mm gesiebt. Das Fibrinogenpulver wird auf einen Feuchtigkeitsgehalt von 0,8 bis 1% gebracht und dann in einem geschlossenen Behälter einem starken Luftstrom bei einer Temperatur von 100° C 30 Minuten lang ausgesetzt. Nach Abkühlung auf Raumtemperatur wird das erhitzte Fibrinogenpulver feuchtigkeitsdicht verschlossen und bei einer Temperatur von 2° bis 8°C gelagert.

### 8. Bestimmung der Rekonstitutionszeit von gefriergetrocknetem Fibrinogen

Unter der "Rekonstitutionszeit" wird im Sinne der vorliegenden Beschreibung und Patentansprüche jene Zeitspanne verstanden, die nach Zugabe des Lösungsmittels zum gefriergetrockneten Fibrinogen bis zu seiner kompletten Auflösung verstreicht, wobei das gefriergetrocknete Fibrinogen dann als gelöst angesehen wird, wenn mindestens 97% in Lösung gegangen sind und nicht mehr als 3% ungelöst verbleiben.

Die Rekonstitutionszeit wird nun wie folgt bestimmt:

Es werden je 500 mg Fibrinogen - bezogen auf gerinnbares Protein - in 6 Zentrifugenbecher eingewogen, mit je 10 ml 0,9%iger Kochsalzlösung versetzt und auf einem Biodancer (New Brunswick Scientific Edison, NJ, USA) bei der Einstellung "Speed 2,0" und bei Zimmertemperatur geschwenkt.

Nach 3, 5, 10, 15, 20 und 30 Minuten wird je ein Zentrifugenglas entnommen und 10 Minuten zentrifugiert, wobei eine Umdrehungszahl von 10.000 Umdrehungen/Minute eingestellt wird. Die in den 6 Zentrifugengläsern enthaltenen Sedimente werden 3x mit 0,9%iger Kochsalzlösung gewaschen und der Proteingehalt der Sedimente nach der dritten Waschung bestimmt. Zur Proteinbestimmung wird das Bradford-Reagenz von Biorad verwendet. Der Proteingehalt wird nach einer Referenzkurve, die mit Hilfe des Bioradreagenz und Fibrinogen erstellt wurde, ermittelt. Die Rekonstitutionszeit in Minuten ist nun jene Zeitspanne nach Zugabe des Lösungsmittels, zu der weniger als 3% des gerinnbaren Proteins ungelöst vorliegen.

Eine schnelle Rekonstitutionszeit ist im Sinne der vorliegenden Beschreibung und Patentansprüche dann gegeben, wenn zur Herstellung von 10 ml einer 5%igen Fibrinogenlösung bei Zimmertemperatur durch leichtes Schwenken ohne Schaumbildung eine Zeitspanne von nicht mehr als 10 Minuten, insbesondere zwischen 3-5 Minuten, benötigt wird.

Die erfindungsgemäß hergestellten, gefriergetrockneten Fibrinogenkonzentrate weisen eine Rekonstitutionszeit von weniger als 10 Minuten, insbesondere zwischen 3-5 Minuten auf. Demgegenüber wurde die Rekonstitutionszeit bei zwei Fibrinogenkonzentraten des Standes der Technik mit 20 Minuten bzw. mit mehr als 30 Minuten gemessen.

## Patentansprüche

1. Verfahren zur Herstellung einer Fibrinogenlösung aus einer wässerigen Lösung, welche funktionell intaktes Fibrinogen enthält und mit Profibrin und/oder Fibrinmonomeren und/oder Fibrinmonomerkomplexen und/oder Fibrinspaltprodukten verunreinigt ist, **dadurch gekennzeichnet, dass** die Verunreinigungen mit einem nicht denaturierenden Fällungsmittel bei einer Temperatur zwischen -4° bis +4°C und einer Ca-Ionenaktivität, die nicht höher ist als jene einer 1.000 µM CaCl₂-Lösung, gefällt und durch Filtration oder Zentrifugation aus der wässerigen Lösung abgetrennt werden.

2. Verfahren nach Anspruch 1, **dadurch gekennzeichnet, dass** die wässerige Lösung bei der Fällung eine Temperatur zwischen 0° und 2°C und eine elektrische Leitfähigkeit von ≤ 500 µS/cm aufweist.

3. Verfahren nach einem der Ansprüche 1 oder 2, **dadurch gekennzeichnet, dass** die Ca-Ionenaktivität nicht höher ist als jene einer 500 µM CaCl₂-Lösung.

4. Verfahren nach Anspruch 3, **dadurch gekennzeichnet, dass** die Ca-Ionenaktivität nicht höher ist als jene einer 50 µM CaCl₂-Lösung.

5. Verfahren nach Anspruch 2, **dadurch gekennzeichnet, dass** die Lösung eine Temperatur zwischen 0° und 2°C, eine elektrische Leitfähigkeit zwischen 10 bis 100 µS/cm und eine Ca-Ionenaktivität aufweist, die nicht höher ist als jene einer 500 µM CaCl₂-Lösung.

6. Verfahren nach einem der Ansprüche 1 bis 5, **dadurch gekennzeichnet, dass** das in der erhaltenen Fibrinogenlösung enthaltene Fibrinogen durch mindestens eine weitere Fällung weiter gereinigt wird.

7. Verfahren nach einem der Ansprüche 1 bis 6 zur Herstellung einer Fibrinogenlösung mit einem Gehalt von 0,3 % bis 10 % gerinnbarem Protein, die bis zu 36 Monate bei einer Temperatur von 2° bis 8°C haltbar ist, durch Abreinigung von Enzymen und deren Proenzymen, die als solche bzw. im aktivierten Zustand Fibrinogen als Substrat erkennen, mit Hilfe von nicht denaturierenden Fällungsmitteln und erforderlicherweise durch Zusatz von entsprechenden virusinaktivierten, homologen Proteaseinhibitoren.

8. Verfahren nach einem der Ansprüche 1 bis 7, **dadurch gekennzeichnet, dass** die Fibrinogenlösung einem Verfahren zur Virusinaktivierung oder Virusabreicherung unterzogen wird, die bei Temperaturen über der Raumtemperatur, einer Thrombinaktivität von ≤ 0,03 IE/ml und einer Ca-Ionenaktivität, die jene einer 1.000 µM CaCl₂-Lösung nicht übersteigt, durchgeführt wird.

9. Verfahren nach Anspruch 8, **dadurch gekennzeichnet, dass** die Viren durch Nanofiltration bei Temperaturen zwischen 20° und 40°C und mit Nanofiltern einer Porenweite von maximal 20 nm, vorzugsweise 15 nm, abgereichert werden, wobei die Nanofiltration vorzugsweise so durchgeführt wird, dass es nicht zur Abspaltung von Fibrinopeptid A kommt.

10. Verfahren zur Herstellung eines Fibrinogenkonzentrats, **dadurch gekennzeichnet, dass** eine nach einem der Ansprüche 1 bis 9 hergestellte Fibrinogenlösung aufkonzentriert, tiefgefroren oder gefriergetrocknet wird.

11. Verfahren nach einem der Ansprüche 1 bis 10, **dadurch gekennzeichnet, dass** die Ca-Ionenaktivität der wässerigen Lösung durch Zusatz von EDTA gesenkt wird und dass als nicht denaturierendes Fällungsmittel Glycin eingesetzt wird.

12. Verfahren nach einem der Ansprüche 2 bis 10 und einem Verfahren nach Anspruch 11, sofern rückbezogen auf einen der Ansprüche 2 bis 10, **dadurch gekennzeichnet, dass** die Absenkung der elektrischen Leitfähigkeit der wässerigen Lösung durch Dialyse, Diafiltration oder Alkoholfällung in der Kälte erreicht wird.

## Claims

1. A method for producing a fibrinogen solution from an aqueous solution containing functionally intact fibrinogen and being contaminated with profibrin and/or fibrin monomers and/or fibrin monomer complexes and/or fibrin split products, **characterized in that** the contaminations are precipitated with a non-denaturing precipitating agent at a temperature between -4° to +4° C and a calcium ion activity not higher than that of a 1,000 µM CaCl₂-solution, and are separated by filtration or centrifugation from the aqueous solution.

2. A method according to claim 1, **characterized in that** the aqueous solution has a temperature between 0° and 2°C and an electric conductivity of ≤ 500 µS/cm when it is precipitated.

3. A method according to any of the preceding claims 1 or 2, **characterized in that** the calcium ion activity is not exceeding that of a 500 µM CaCl₂ solution.

4. A method according to claim 3, **characterized in that** the calcium ion activity is not exceeding than that of a 50 µM CaCl₂ solution.

5. A method according to claim 2, **characterized in that** the solution has a temperature between 0° and 2° C, an electric conductivity between 10 to 100 µS/cm and a calcium ion activity not exceeding that of a 500 µM CaCl₂ solution.

6. A method according to any of the claims 1 to 5, **characterized in that** the fibrinogen contained in the obtained fibrinogen solution is further purified by at least one more precipitation step.

7. A method according to any of the claims 1 to 6 for the production of a fibrinogen solution containing 0.3% to 10% clottable protein, which may be stored up to 36 months at a temperature of 2° to 8° C, by removal of enzymes and their pro-enzymes which, as such or in activated form, recognize fibrinogen as a substrate, using non-denaturing precipitating agents and, if necessary, by additing appropriate virus inactivated, homologous protease inhibitors.

8. A method according to any of the claims 1 to 7, **characterized in that** the fibrinogen solution is subjected to a method for virus inactivation or virus removal, which is carried out at temperatures above room temperature, a thrombin activity of ≤ 0.03 IE/ml and a calcium ion activity not exceeding that of a 1,000 µM CaCl₂ solution.

9. A method according to claim 8, **characterized in that** the viruses are removed by means of nanofiltration at temperatures between 20° and 40° C and using nanofilters with a pore size of at most 20 nm, preferably 15 nm, said nanofiltration preferably being carried out in a way so that fibrinopeptide A is not split off.

10. A method for producing a fibrinogen concentrate, **characterized in that** a fibrinogen solution produced according to any of the claims 1 to 9 is concentrated, deep-frozen or freeze-dried.

11. A method according to any of the claims 1 to 10, **characterized in that** the calcium ion activity of the aqueous solution is lowered by adding EDTA and that glycine is used as non-denaturating precipitating agent.

12. A method according to any of the claims 2 to 10 and a method according to claim 11 as far as it is dependent on one of the claims 2 to 10, **characterized in that** the reduction of electric conductivity of the aqueous solution is realized by dialysis, diafiltration or alcohol precipitation in the cold.

## Revendications

1. Procédé servant à fabriquer une solution de fibrinogène à partir d'une solution aqueuse, qui contient un fibrinogène intact fonctionnellement et est contaminée de profibrine et/ou de monomères de fibrine et/ou de complexes monomères de fibrine et/ou de produits de clivage de fibrine, **caractérisé en ce que** les impuretés sont précipitées à l'aide d'un moyen de précipitation non dénaturant à une température comprise entre -4° et +4°C et avec une activité des ions de Ca qui n'est pas supérieure à l'activité d'une solution de 1000 µM de CaCl₂, et **en ce qu'**elles sont éliminées par filtration ou centrifugation de la solution aqueuse.

2. Procédé selon la revendication 1, **caractérisé en ce que** la solution aqueuse présente, lors de la précipitation, une température comprise entre 0° et 2 °C et une conductivité électrique ≤ 500 µS/cm.

3. Procédé selon l'une quelconque des revendications 1 ou 2, **caractérisé en ce que** l'activité des ions de CA n'est pas supérieure à l'activité d'une solution de 500 µM de CaCl₂.

4. Procédé selon la revendication 3, **caractérisé en ce que** l'activité des ions de Ca n'est pas supérieure à l'activité d'une solution de 50 µM de CaCl₂.

5. Procédé selon la revendication 2, **caractérisé en ce que** la solution présente une température comprise entre 0° et 2 °C, une conductivité électrique comprise entre 10 et 100 µS/cm et une activité des ions Ca, qui n'est pas supérieure à l'activité d'une solution de 500 µM de CaCl₂.

6. Procédé selon l'une quelconque des revendications 1 à 5, **caractérisé en ce que** le fibrinogène contenu dans la solution de fibrinogène obtenue est davantage purifié par au moins une précipitation supplémentaire.

7. Procédé selon l'une quelconque des revendications 1 à 6 servant à produire une solution de fibrinogène comprenant une teneur allant de 0,3 % à 10 % de protéine coagulable, qui peut être conservée jusqu'à 36 mois à une température allant de 2° à 8 °C, par l'élimination d'enzymes et des proenzymes de ces dernières, qui en tant que telles ou dans l'état activé identifient le fibrinogène comme substrat, à l'aide de moyens de précipitation non dénaturants, et au besoin par l'addition d'inhibiteurs de protéases correspondants à virus inactivés, homologues.

8. Procédé selon l'une quelconque des revendications 1 à 7, **caractérisé en ce que** la solution de fibrinogène est soumise à un procédé servant à l'inactivation de virus ou la clairance virale, qui est effectué à des températures supérieures à la température ambiante, en présence d'une activité de thrombine ≤ 0,03 IE/ml et d'une activité des ions Ca, qui ne dépasse pas l'activité d'une solution de 1000 µm de CaCl₂.

9. Procédé selon la revendication 8, **caractérisé en ce que** les virus sont soumis à une clairance par nanofiltration à des températures comprises entre 20° et 40 °C et à l'aide de nanofiltres d'une largeur de pore de 20 nm au maximum, de préférence de 15 nm, sachant que la nanofiltration est effectuée de préférence de manière à ne pas aboutir à un clivage de fibrinopeptide A.

10. Procédé servant à produire un concentré de fibrinogène, **caractérisé en ce qu'**une solution de fibrinogène produite selon l'une quelconque des revendications 1 à 9 est concentrée, surgelée ou lyophilisée.

11. Procédé selon l'une quelconque des revendications 1 à 10, **caractérisé en ce que** l'activité des ions Ca de la solution aqueuse est abaissée par l'addition d'EDTA, et **en ce qu'**on utilise comme moyen de précipitation non dénaturant de la glycine.

12. Procédé selon l'une quelconque des revendications 2 à 10 et procédé selon la revendication 11, en lien avec l'une quelconque des revendications 2 à 10, **caractérisé en ce que** l'abaissement de la conductivité électrique de la solution aqueuse est obtenu à froid par dialyse, diafiltration ou précipitation à l'alcool.
